Europäisches Patentamt

European Patent Office

Office européen des brevets

(19)

(11) EP 0 980 671 A1

(12) EUROPÄISCHE PATENTANMELDUNG

(43) Veröffentlichungstag:
23.02.2000  Patentblatt 2000/08

(51) Int. Cl.⁷: A61B 6/08, G03B 42/02

(21) Anmeldenummer: 99202612.0

(22) Anmeldetag: 10.08.1999

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(30) Priorität: 19.08.1998 DE 19837512

(71) Anmelder:
• Philips Corporate Intellectual Property GmbH
52064 Aachen (DE)
Benannte Vertragsstaaten:
DE

• Koninklijke Philips Electronics N.V.
5621 BA  Eindhoven (NL)
Benannte Vertragsstaaten:
FR GB NL

(72) Erfinder: Kunert, Heinz-Peter,
22335 Hamburg (DE)

(74) Vertreter:
Hartmann, Heinrich et al
Philips Corporate Intellectual Property GmbH,
Habsburgerallee 1
52064 Aachen (DE)

(54) **Röntgenuntersuchungsgerät mit einer Blendeneinheit**

(57)   Die Erfindung bezieht sich auf ein Röntgenuntersuchungsgerät mit einem Röntgenstrahler (2) und einer mit dem Röntgenstrahler verbundenen Blendeneinheit (4), die mit Blenden zur Ausblendung eines vom Brennfleck (21) des Röntgenstrahlers ausgehenden Strahlenkegels (210) und mit einer Lichtquelle (42) zur Erzeugung eines über einen Spiegel (43) durch die Blenden (41) hindurchtretenden Lichtkegels (420) versehen ist. Wenn die Abmessungen der lichtemittierenden Teile der Lichtquelle wesentlich größer sind als der Brennfleck, ist das vom Strahlenkegel bestrahlte Strahlenfeld kleiner als das vom Lichtkegel beleuchtete Lichtfeld. Um das Strahlenfeld und das Lichtfeld in Übereinstimmung zu bringen, ist vorgesehen, daß die Blenden mit für Röntgenstrahlung transparenten Korrektur-Blenden (44) versehen sind, die lichtundurchlässig sind und den Lichtkegel begrenzen.

FIG. 1

EP 0 980 671 A1

**Beschreibung**

Die Erfindung betrifft ein Röntgenuntersuchungsgerät mit einem Röntgenstrahler und einer mit dem Röntgenstrahler verbundenen Blendeneinheit, die mit Blenden zur Ausblendung eines von einem Brennfleck des Röntgenstrahlers ausgehenden Strahlenkegels und mit einer Lichtquelle zur Erzeugung eines über eine Reflektoranordnung durch die Blenden hindurchtretenden Lichtkegels versehen ist. Außerdem bezieht sich die Erfindung aufeine Blendeneinheit für einen Röntgenstrahler. Ein solches Röntgenuntersuchungsgerät und eine solche Blendeneinheit sind aus der US-Patentanmeldung 08/808,594 (PHD 96-032 US) bekannt.

Der Lichtkegel dient dem Benutzer dabei als Einstellhilfe, die ihm die Einstellung des bei einer nachfolgenden Röntgenaufnahme von der Röntgenstrahlung getroffenen Strahlenfeldes erleichtert. Der Lichtkegel beleuchtet dabei entweder den zu untersuchenden Patienten oder - bei einem Rönrgenuntersuchungsgerät mit einer Rasterlade - die Rasterlade, wenn der Röntgenstrahler und die Rasterlade mit dem zu belichtenden Film vorübergehend seitlich neben die Tischplatte gezogen werden, auf der sich der Patient befindet. Die beleuchtete Fläche sollte dabei mit der Fläche übereinstimmen, die bei einer nachfolgenden Röntgenaufnahme vom Strahlenkegel des Röntgenstrahlers getroffen wird.

Die Lichtquelle hat von den Blendenplatten dabei den gleichen optischen Abstand, wie der Brennfleck der Strahlenquelle. Die Lichtquelle soll möglichst billig sein, eine hohe Lebensdauer und eine große Helligkeit aufweisen, und der von ihr ausgeblendete Lichtkegel soll möglichst genau mit dem Strahlenkegel übereinstimmen, der bei einer nachfolgenden Röntgenaufnahme erzeugt wird. Lichtquellen, d.h. die das Licht emittierende Struktur, z.B die Glühwendel, der Lichtquelle hat aber im Vergleich zum Brennfleck des Rönrgenstrahlers große Abmessungen. Infolgedessen ist die von der Lichtquelle beleuchtete Fläche größer als die anschließend von Röntgenstrahlung getroffene Fläche.

Um die vom Röntgenstrahler bestrahlte Fläche und die von der Lichtquelle beleuchtete Fläche in Übereinstimmung zu bringen, ist bei dem bekannten Röntgenuntersuchungsgerät ein Motorantrieb der Blenden vorgesehen, der so gesteuert wird, daß die Blendenöffnung bei der Einstellung mit der Lichtquelle kleiner ist als bei einer anschließenden Röntgenaufnahme. Diese Lösung setzt das Vorhandensein eines entsprechend gesteuerten Motorantriebs voraus.

Aufgabe der vorliegenden Erfindung ist es, ein Röntgenuntersuchungsgerät sowie eine Blendeneinheit zu schaffen, die keinen Motorantrieb benötigen, um das Lichtfeld und das Strahlenfeld in Übereinstimmung zu bringen. Diese Aufgabe wird erfindungsgemäß dadurch gelöst, daß die Blenden mit für Röntgenstrahlung transparenten Korrektur-Blenden versehen sind, die lichtundurchlässig sind und den Lichtkegel begrenzen. Eine entsprechend ausgebildete Blendeneinheit ist in Anspruch 6. beschrieben.

Die erfindungsgemäßen Korrektur-Blenden haben aufden Strahlenkegel keinen Einfluß, weil sie für die Röntgenstrahlung transparent sind, sie engen aber den Lichtkegel ein. Bei geeigneter Bemessung lassen sich somit das Lichtfeld und das Stahlenfeld miteinander in Übereinstimmung bringen. Exakt kann man diese Übereinstimmung nur für einen definierten Abstand zwischen dem Brennfleck und dem Bildwandler (z.B. einem Film) erreichen. Für andere Abstände (bei Bucky-Aufnahmegeräten arbeitet man in der Regel mit einem definierten Abstand - z.B. 1,15 m) gilt diese Übereinstimmung nicht mehr exakt, sie ist aber wesentlich besser als ohne die Korrektur-Blenden.

Einfache Röntgenuntersuchungsgeräte können eine oder mehrere unterschiedliche Blendenöffnungen haben, von denen jeweils eine den Strahlenkegel begrenzt. In diesem Fall müssen die Korrektur-Blenden die Blendenöffnungen allseits einengen. Moderne Röntgenuntersuchungsgeräre besitzen aber zwei vorzugsweise verstellbare Blendenplattenpaare zur Begrenzung des Strahlenkegels in zwei zueinander senkrechten Richtungen. Anspruch 2 beschreibt eine für solche Röntgenuntersuchungsgeräte geeignete Ausgestaltung der Erfindung. Die Korrektur-Blenden können in diesem Fall in verschiedener Weise angeordnet sein:

Eine erste Möglichkeit beschreibt Anspruch 3. Dabei befinden sich die Korrektur-Blenden bei jedem Blendenplattenpaar aufderselben Seite. Allerdings verhindern die Korrektur-Blenden in diesem Fall, daß das Blendenplattenpaar vollständig geschlossen wird. Dies ist in der Regel auch nicht erforderlich.

Eine andere Möglichkeit ist in Anspruch 4 beschrieben, die eine vollständige Schließung der Blendenplattenpaare erlaubt, weil sie in verschiedenen Ebenen liegen. Eine weitere Ausgestaltung dieser letzteren Alternative ist in Anspruch 5 beschrieben. Damit lassen sich die Unsymmetrien ausgleichen, die sich bei einer Röntgenröhre mit Strichfokus für eines der beiden Blendenplattenpaare ergeben.

Die Erfindung wird nachstehend anhand der Zeichnungen näher erläutert. Es zeigen:

Fig. 1 Ein erfindungsgemäßes Bucky-Röntgenuntersuchungsgerät,

Fig. 2 den Lichtkegel und den Strablenkegel bei einem solchen Röntgenuntersuchungsgerät und

Fig. 3 verschiedene Anordnungen der Korrektur-Blenden an den Blendenplatten.

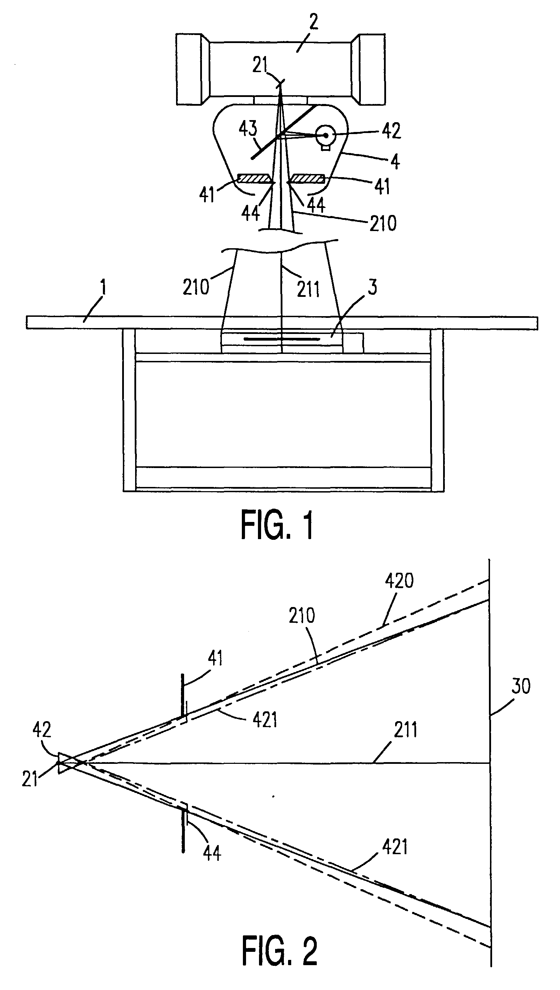

Fig. 1 zeigt ein Bucky-Röntgenuntersuchungsgerät, das einen Patientenlagerungstisch 1 und einen Röntgenstrahler 2 umfaßt. Unterhalb der Tischplatte des Patientenlagerungstisches 1 befindet sich ein in Längsrichtung der Tischplatte verschiebbares Laufraster 3 mit einem bei einer Röntgenaufnahme zu belichtenden Film. Der Röntgenstrahler 2 wird von einem nicht näher dargestellten Stativ getragen, das in Tischlängsrichtung verschiebbar ist, wobei er von einer Position, in der sein Strahlenkegel aufdie Mitte der Tischplatte auftrifft, senkrecht zur Zeichenebene in eine Position verschiebbar ist, in der es sich senkrecht oberhalb des unter der Tischplatte hervorgezogenen Laufrasters 3 befindet. In dieser Stellung kann eine Einblendung auf das gewünschte Aufnahmeformat erfolgen.

Die Einblendung erfolgt mittels einer Blendeneinheit 4, die am Röntgenstrahler 2 befestigt ist. Die Röntgenstrahlung, die von dem Brennfleck bzw. Fokus 21 einer nicht näher dargestellten, im Röntgenstrahler 2 befindlichen Röntgenröhre ausgeht, wird durch ein erstes Blendenplattenpaar 41 mit zueinander parallelen und zur Zeichenebene der Fig. 1 senkrechten Blendenkanten begrenzt. Außerdem ist noch ein zweites, nicht näher dargestelltes Blendenplattenpaar vorgesehen, das horizontale, parallel zur Zeichenebene verlaufende Blendenkanten aufweist und das die Röntgenstrahlung in Richtung senkrecht zur Zeichenebene begrenzt. Der durch die Blendenplatten begrenzte Strahlenkegel ist mit 210 bezeichnet; der Strahlenkegel ist durch die geschlängelten Linien unterbrochen, weil der Abstand des Röntgenstrahlers 2 in der Praxis größer ist als in Fig. 1 dargestellt. Der Strahl im Zentrum des Strahlenkegels - der sogenannte Zentralstrahl - ist mit 211 bezeichnet. Er trifft senkrecht auf das Laufraster 3 in dessen Zentrum auf.

In der Blendeneinheit 4 befindet sich eine Lichtquelle 42, die über einen - für die Röntgenstrahlung transparenten - Spiegel 43 und durch die Blendenplatten 41 hindurch die Tischplatte (bzw. einen darauf befindlichen Patienten) beleuchtet. Die Lichtquelle 42 hat denselben Abstand vom Spiegel 43 wie der Brennfleck 21. Wenn sie (bzw. die in ihr enthaltene lichtemittierende Struktur) die gleichen Abmessungen hätte, wie der Brennfleck 21, müßte daher der durch die Blendenplatten 41 ausgeblendete Lichkegel mit dem Strahlenkegel 210 übereinstimmen und infolgedessen auch das Lichtfeld (d.h. der Querschnitt des Lichtkegels in der Ebene des im Laufraster 3 enthaltenen Films) mit dem Strahlenfeld (dem Querschnitt des Strahlenkegels 210 in der Ebene des im Laufraster 3 befindlichen Films).

Aus den eingangs genannten Gründen ist die Licht emittierende Struktur der Lichtquelle (z.B. die Wendel einer Glühlampe) deutlich größer als der Brennfleck. In der Praxis können die Abmessungen der Wendel z.B. 6 x 3,2 mm betragen, während die (optisch wirksame) Größe des Brennflecks 1 x 1 mm - oder weniger - beträgt. Bei einer derart ausgedehnten Wendel ist der Kernschattenbereich (das ist der Bereich auf den keinerlei Licht gelangt) deutlich größer als der von sämtlichen Licht emittierenden Punkten der Lichtquelle getroffene Bereich. Die Kante des Lichtfeldes liegt dabei - relativ gut reproduzierbar - bei ca. 8% der Helligkeit im Zentrum. Infolgedessen ist auch das vom Benutzer wahrnehmbare Lichtfeld deutlich größer als das vollständig beleuchtete Feld, das im wesentlichen mit dem Strahlenfeld übereinstimmt.

Der durch die Differenz dieser Felder definierte Abbildungsfehler beträgt bei den angegebenen Abmessungen der Wendel und bei 1 m Abstand zwischen dem Film und dem Fokus 21 in Richtung der größeren Wendelbreite ca. 15 mm und bei einem Abstand von 2 m ca 35 mm. Um diesen Abbildungsfehler zu verringern bzw. zu beseitigen, sind die Blendenplanen 41 mit Korrektur-Blenden 44 versehen, deren Kanten parallel zu den Kamen der Blendenplatten 41 verlaufen und die den Lichtkegel einengen. Da die Korrektur-Blenden aber aus einem für Röntgenstrahlung transparenten Material bestehen, wird der Strahlenkegel 210 davon nicht beeinflußt.

Die Wirkung der Korrektur-Blenden wird anhand von Fig. 2 erläutert, wobei der Zentralstrahl 211 allerdings nicht vertikal verläuft wie bei Fig. 1 sondern horizontal. Dabei ist die Lichtwendel 42 besonders groß angenommen, um die Effekte besser hervortreten zu lassen. Außerdem ist der einfacheren Darstellbarkeit halber angenommen, daß sich die Lichtquelle 42 am Ort des Brennflecks 21 befindet; da der optische Abstand zwischen der Wendel 42 und dem Film aber genauso groß ist, wie der Abstand zwischen Brennfleck und Film, verfälscht diese Annahme die tatsächlichen Verhältnisse nicht.

Der vom Brennfleck 21 ausgehende Strahlenkegel 210 ist mit ausgezogenen Linien dargestellt. Mit gestrichelten Linien ist der Lichtkegel 420 dargestellt, der sich ergeben würde, wenn die Korrektur-Blenden 44 nicht vorhanden wären. Man erkennt deutlich, daß das Lichtfeld in der Ebene des im Laufraster befindlichen Films 30 größer ist als das Strahlenfeld. Die Korrektur-Blenden 44 bewirken eine Einengung des Lichtkegels, so daß der mit strichpunktierten Linien angedeutete Lichtkegel 421 resultiert. Bei geeigneter Breite der Korrekturblenden (mit "Breite" ist in diesem Fall der Abstand der das Licht begrenzenden Kante der Korrektur-Blende 44 von der die Röntgenstrahlung begrenzenden Kante der Blendenplatte 41 bezeichnet) läßt sich das Lichtfeld mit dem Strahlenfeld in Übereinstimmung bringen.

Für die Breite b der Korrektur-Blende gilt in guter Näherung die Gleichung

$$b = \frac{1}{2}(B_s - B_x)(1 - {}^d/_D) \qquad (1)$$

Dabei ist $B_s$ die Breite der Lichtquelle, $B_x$ die Brennfleckbreite, d der Abstand der Ebene der Blendenkanten vom Brennfleck und D der Abstand des Brennflecks vom Film 30 (Film-Fokus-Abstand).

Mit den aus der Praxis genommenen Werten d

= 0,25 m, $B_s$ = 5 mm und $B_x$ = 1 mm ergibt sich daraus die Breite der Korrektur-Blende zu 1,5 mm für einen Film-Fokus Abstand D von 1m und zu 1,75 mm für einen Abstand D von 2 m. Wenn die Korrektur-Blende 44 starr mit der Blendenplane 41 verbunden ist, ist die Blendenbreite B konstant. Für einen Wen B = 1,5 mm wird der Abbildungsfehler bei D = 1m vollständig korrigiert (ohne Korrektur-Blende würde er 12 mm betragen). Bei einem Film-Fokus Abstand D = 2 m wird der Abbildungsfehler von 28 mm auf 3,5 mm reduziert. Das kleinste noch einstellbare Aufnahmeformat beträgt in diesem Beispiel 12 mm bei einem Film-Fokus Abstand D von 1 m. Dieser Wert ist für die Praxis völlig ausreichend; die gesetzmäßig einzuhaltende maximale Abweichung zwischen Lichtfeld und Strahlenfeld von 20 mm bei einem Film-Fokus Abstand D von 1 m wird dabei deutlich unterschritten.

Man kann die Breite der Korrektur-Blende auch für einen Wert D zwischen 1 m und 2 m bemessen. Der maximale Abbildungsfehler wäre dann kleiner als im angenommenen Fall. In der Regel wird aber eine genaue Einblendung bei dem Standardabstand (1m, neuerdings auch 1,15m) angestrebt.

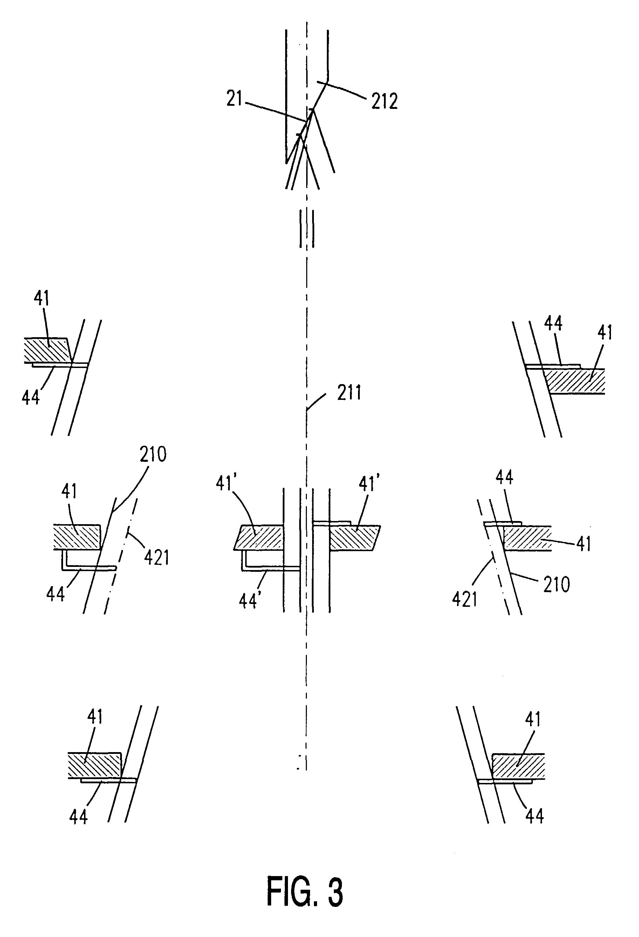

Fig. 3 zeigt in schematischer Darstellung verschiedene Möglichkeiten der Anordnung der Korrektur-Blenden. Dabei ist mit 212 der Anodenteller einer Drehanoden-Röntgenröhre bezeichnet, bei der der Brennfleck 21 aufeiner zur horizontalen Rotationsachse des Anodentellers 212 konzentrischen Brennfleckbahn angeordnet ist. Die Brennfleckbahn hat eine Neigung von 10° bis 20° in bezug auf die Vertikale, und die Abmessungen des Brennflecks 21 sind nach dem Prinzip des Strichfokus in Richtung senkrecht zur Brennfleckbahn größer als in Richtung der Brennfleckbahn, so daß - vom Zentralstrahl 211 aus gesehen - der Brennfleck als quadratisch erscheint. Da die Ebene der Brennfleckbahn sich von links unten nach rechts oben neigt, erscheint der Brennfleck von der rechten Blendenkante aus gesehen größer als von der linken Blendenkante aus. D.h. die Breite $B_x$ des Brennflecks erscheint an der rechten Seite des Strahlenfeldes größer als an der linken Seite.

In Fig. 3 sind in drei unterschiedlichen Zeilen die verschiedenen Anordnungsmöglichkeiten der Korrektur-Blenden 44 dargestellt. In der untersten Zeile befinden sich die Korrektur-Blenden jeweils unterhalb der Blendenplatten 41, und sie haben die gleiche Breite. Diese Anordnung erfordert den geringsten Herstellungs- und Montageaufwand, hat aber den Nachteil, daß die Blende nicht so weit geschlossen werden kann, daß keine Röntgenstrahlung mehr durchdringt, weil ein Schließen der Blenden nicht mehr möglich ist, sobald die einander zugewandten Blendenkanten der Korrektur-Blenden 44 einander berühren. An sich ist ein vollständiges Schließen der Blendenplatten für die Praxis auch nicht erforderlich. Ggf. könnten aber die Korrektur-Blenden 44 federnd mit den Blendenplatten 41 verbunden sein, so daß die Korrektur-Blenden beim Schließen entgegen der Federkraft verschoben werden.

In der obersten der drei Zeilen in Fig. 3 ist eine andere Anordnung der Korrektur-Blenden 44 dargestellt. Die eine Korrektur-Blende 44 ist dabei an der Unterseite der linken Blendenplatte 41 befestigt, und die andere Korrektur-Blende ist fest mit der Oberseite der rechten Blendenplatte 41 verbunden. In diesem Fall wird das vollständige Schließen der Blendenplatten für die Röntgenstrahlung nicht durch die Korrekturblenden verbinden.

In der zweiten Zeile ist eine zu der oberen Zeile ähnliche Anordnung dargestellt, wobei eine der beiden Korrektur-Blenden - im Beispiel die linke untere - von der zugehörigen Blendenplatte einen gewissen Abstand hat. Für die linke wie für die rechte Seite sind der Randstrahl des Strahlenkegels 210 (ausgezogene Linien) und des Lichtkegels 421 (strichpunktierte Linien) dargestellt.

Man erkennt, daß diese Randstrahlen bei der rechten Blendenplatte enger beisammenliegen als bei der linken Blendenplatte. Infolgedessen ist die Korrektur des Lichtkegels durch die Korrektur-Blende 44 auf der rechten Seite schwächer als auf der linken Seite, was nach Gleichung 1 auch erforderlich ist, weil der Brennfleck von der rechten Blendenkante aus größer erscheint als von der linken Blendenkante aus. Die durch die unsymmetrische Lage der Brennfleckbahn in bezug aufdie beiden Blendenkanten hervorgerufene Unsymmetrie wird dadurch also kompensiert. Schließt man die Blendenplatten jedoch, so daß die Blendenplatten und die Korrektur-Blenden, die mit 41' bzw. 44' bezeichneten Stellungen einnehmen, dann verringert sich die Unsymmetrie, so daß die Randstrahlen des Lichtkegels bzw. des Strahlenkegels symmetrisch zum Zentralstrahl 211 sind.

**Patentansprüche**

1. Röntgenuntersuchungsgerät mit einem Röntgenstrahler (2) und einer mit dem Röntgenstrahler verbundenen Blendeneinheit (4), die mit Blenden (41) zur Ausbiendung eines von einem Brennfleck des Röntgenstrahlers ausgehenden Strahlenkegels (210) und mit einer Lichtquelle (42) zur Erzeugung eines über eine Reflektoranordnung (43) durch die Blenden hindurchtretenden Lichtkegels versehen ist,
   dadurch gekennzeichnet, daß die Blenden (41) mit für Röntgenstrahlung transparenten Korrektur-Blenden (44) versehen sind, die lichtundurchlässig sind und den Lichtkegel (421) begrenzen.

2. Röntgenuntersuchungsgerät nach Anspruch 1, dadurch gekennzeichnet, daß die Blenden zwei vorzugsweise verstellbare Paare von Blendenplatten (41) mit paarweise parallelen Blendenkanten zur Begrenzung des Strahlenkegels in zwei zueinander senkrechten Richtungen aufweisen und daß an jeder Blendenplatte eine Korrektur-Blende (44) mit ihrer Kante parallel zur Blendenkante befestigt ist.

**3.** Röntgenuntersuchungsgerät nach Anspruch 2, dadurch gekennzeichnet, daß die mit einem Blendenplatten-Paar (41) verbundenen Korrektur-Blenden (44) sich in der in der gleichen Ebene befinden.

**4.** Röntgenuntersuchungsgerät nach Anspruch 2, dadurch gekennzeichnet, daß die mit einem Blendenplatten-Paar (41) verbundenen Korrekturblenden sich in gegeneinander versetzten Ebenen befinden.

**5.** Röntgenuntersuchungsgerät nach Anspruch 4, dadurch gekennzeichnet, daß der Brennfleck (21) des Röntgenstrahlers (2) gegenüber den Ebenen der Korrekturblenden geneigt ist, und daß der Versatz zwischen den Ebenen der Korrekturblenden so gewählt ist, daß die durch die Neigung bedingte Unsymmetrie des Strahlenkegels (210) durch eine entsprechende Unsymmetrie des Lichtkegels (421) wenigstens teilweise kompensiert wird.

**6.** Blendeneinheit für einen Röntgenstrahler, die mit Blenden(41) zur Ausblendung eines von einem Brennfleck (21) des Röntgenstrahlers ausgehenden Strahlenkegels (210) und mit einer Lichtquelle (42) zur Erzeugung eines über eine Reflektoranordnung durch die Blenden hindurchtretenden Lichtkegels versehen ist
dadurch gekennzeichnet, daß die Blenden (41) mit für Röntgenstrahlung transparenten Korrektur-Blenden (44) versehen sind, die lichtundurchlässig sind und den Lichtkegel (421) begrenzen.

FIG. 1

FIG. 2

21 — 212

41
44

44 — 41

210
41
421

41' — 41'
44'

44 — 41
421 — 210

211

41
44

41
44

# FIG. 3

Europäisches
Patentamt

**EUROPÄISCHER RECHERCHENBERICHT**

Nummer der Anmeldung

EP 99 20 2612

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | PATENT ABSTRACTS OF JAPAN vol. 1995, no. 9, 31. Oktober 1995 (1995-10-31) & JP 07 148159 A (SHIMADZU CIRP.), 13. Juni 1995 (1995-06-13) * Zusammenfassung * | 1-3,6 | A61B6/08 G03B42/02 |
| A | US 3 921 001 A (P.R.EDHOLM) 18. November 1975 (1975-11-18) * Spalte 2 - Spalte 3; Abbildungen 1-5 * | 1 | |
| A | US 4 670 896 A (R.KLAUSZ) 2. Juni 1987 (1987-06-02) * Spalte 2 - Spalte 4; Abbildungen 1,2 * | 1 | |

RECHERCHIERTE SACHGEBIETE (Int.Cl.7)

A61B
G03B

Der vorliegende Recherchenbericht wurde für alle Patentansprüche erstellt

| Recherchenort | Abschlußdatum der Recherche | Prüfer |
|---|---|---|
| DEN HAAG | 11. November 1999 | Boeykens, J |

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 99 20 2612

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-11-1999

| Im Recherchenbericht angeführtes Patentdokument | Datum der Veröffentlichung | Mitglied(er) der Patentfamilie | Datum der Veröffentlichung |
|---|---|---|---|
| JP 07148159 A | 13-06-1995 | KEINE | |
| US 3921001 A | 18-11-1975 | FR 2197299 A<br>GB 1388342 A | 22-03-1974<br>26-03-1975 |
| US 4670896 A | 02-06-1987 | FR 2561516 A<br>DE 3561786 A<br>EP 0157688 A | 27-09-1985<br>14-04-1988<br>09-10-1985 |

EPO FORM P0461

Für nähere Einzelheiten zu diesem Anhang : siehe Amtsblatt des Europäischen Patentamts, Nr. 12/82